# EUROPEAN PATENT APPLICATION

(11) **EP 4 570 898 A1**
(43) Date of publication of application: **18.06.2025**
(21) Application number: 23216809.6
(22) Date of filing: 14.12.2023
(51) Int. Cl.: C12M 1/00

(54) **METHOD FOR THE RECOVERY OF A BIOCATALYTICALLY PRODUCED PRODUCT FROM AN ORGANIC FLUID STREAM**

(71) Applicant: Delft Advanced Biofuels B.V., 2613 AX Delft (NL)
(72) Inventor: OUDSHOORN, Arjan, Delft (NL); VAN LOTRINGEN, Marion, Delft (NL)
(74) Representative: V.O.

(57) **Abstract**

The invention relates to a method for the recovery of a biocatalytically produced organic product from a fluid stream (S0) originating from a biocatalytic reactor, the fluid stream comprising
an organic liquid phase (L1), comprising the product;
an aqueous liquid phase (L2);
solid matter (SM),
comprising
a) removing solid matter from S0, L1, L2 or from an organic fluid stream (S1) obtained in (b);
b) separating L2 at least substantially off from S0, thereby forming an organic fluid stream (S1), which organic fluid stream (S1) is enriched in biocatalytically produced product, compared to the fluid stream (S0) originating from the biocatalytic reactor; and
c) if desired, after removing solid matter and separating the aqueous liquid phase (L2) at least substantially from the organic liquid phase (L1), recovering the biocatalytically produced product from the organic fluid stream (S1).

## Description

The present disclosure relates to a method for product recovery from a fluid stream (S0) originating from a biocatalytic reactor system.

There is a strong world-wide effort to provide more sustainable production routes, e.g. transition from fossil based substances to bio-based substances, for production of various organic substances, e.g. alcohols, esters, amino acids, carbohydrates, ethers, lipids, ketones, aldehydes, organic acids, pyridines. These substances can be used in a variety of applications for example as flavours, fragrances, cosmetic or food ingredients, nutraceutical products, bioinsecticides, specialty or commodity chemicals, or as biofuels, all as either sustainable replacement or as novel more sustainable product.

These more sustainable production routes include amongst others biocatalytic processes. It is known in the art to produce organic substances by biocatalytic processes such as fermentation and cell free conversions. In such a fermentation process, micro-organisms are used to convert a suitable substrate into an organic substance of interest. In a cell free conversion, enzymes convert a substrate into an organic substance of interest.

More specifically in the world wide effort to produce substances more sustainably there is a need to make more lipophilic products via biocatalysis. Most lipophilic products typically have limited solubility in water due to their 'oily' nature and some are toxic/inhibiting to micro-organisms. These lipophilic products and/or by-products can be inhibiting or toxic to the micro-organism, the products can be unstable, can be unstable under the reaction conditions in the overall reaction vessel or can stick to the micro-organism or any other surface. These effects create challenges for industrial production of these products and hamper the transition towards more sustainable industrial bio-based production. The production per process volume will be low resulting in high production cost per unit of product, creating competition disadvantage for bio-based production routes. Lower product concentrations, i.e. relatively large stream sizes, lead to large reactor volumes and increased effort needed to perform product recovery and/or purification to end product. Additionally biocatalytic processes can generate high amount and variety of chemicals/compounds (often hundreds of chemicals) that make product recovery and further purification complex and costly. Additionally, different feed stocks may be used in the search for a cheaper carbon source. Second and third generation biomass may contain contaminants, which can be inhibiting and/or toxic to the micro-organism as well. They pose a limit on how efficient a biocatalytic process can be related to the overall production rate possible and also on product concentration being reached.

It is known in the art that biocatalytic production systems, such as fermentation production systems, experiencing diluted product streams, inhibition, equilibrium limited, and/or product stability constrained systems benefit from in situ or integrated product removal. Product removal can occur through a continuous phase in and out flow. Often this has the form of a gas stripping or liquid phase extraction, depending on product volatility.

For the liquid phase extraction an auxiliary liquid phase may be used to extract the product from the aqueous biocatalytic phase, preferably green solvents, non-hazardous and non-toxic. This auxiliary liquid phase is typically a compound or mixture of compounds that is at least substantially insoluble in water. The product recovery phase is usually chosen based on the organic substance that is to be recovered, i.e. it is a phase for which the produced substance has a higher affinity than for the aqueous phase in which the product recovery phase is dispersed. Thus, the organic substance of interest is removed from the aqueous phase by phase affinity difference. Suitable phases can be selected for an organic substance of interest based on common general knowledge or empirically. A further property to consider when choosing the product recovery phase is the density of the product recovery phase. A difference in density with the aqueous phase is desirable because this allows separation by gravitation or centrifuging.

Not readily utilized in industry, but particularly suitable liquids for the auxiliary liquid phase are organic liquids, in particular hydrophobic organic liquids, which form a phase separate from the aqueous phase of the reaction mixture.

As more and more biocatalytic processes start using this auxiliary liquid phase for liquid-liquid extraction for substance (e.g. product) removal from the aqueous stream originating from biocatalytic processes, there is a need for substance (e.g. product) recovery from this auxiliary liquid phase.

The typical biocatalytic separation processes, i.e. downstream processing, are water based processes, focussed on recovering substances in very low concentrations from large fractions of water, e.g. evaporation of water, chromatography, ultrafiltration. These processes are cost intensive, both as result of the technique used as well as the low concentration of substance to be recovered resulting in low driving force for separation.

WO2021/010822 describes a way to produce organic substances biocatalytically in an economically feasible manner by using an integrated multiphase reactor system, wherein in a reaction section an organic substance is biocatalytically produced in an aqueous reaction mixture and wherein the produced organic substance is transferred to a product recovery phase or forms a product recovery phase itself, and which product recovery phase is dispersed in the aqueous reaction mixture. The system further comprises a separation section, wherein the product recovery phase is separated from the aqueous reaction mixture. WO2021/010822 further describes that using an auxiliary liquid phase with a boiling point higher than water, to extract the product from the aqueous biocatalytic phase is beneficial for downstream processing, especially from a stream size/concentration and energy utilization perspective. Especially the combination of the boiling point of the auxiliary liquid in combination with the product is of interest for the chosen product recovery route. In WO2021/010822 product recovery is described from an substantially aqueous free organic liquid phase, without need for aqueous phase or solid content removal.

The inventors realized that there remains a need for a method for downstream processing for product recovery from a fluid stream (S0), bioreactor system effluent stream, sometimes referred to as product recovery or harvest stream. More specifically there is a need for a downstream processing methodology for a fluid stream (S0) enriched in organic substance(s), including biocatalytically produced product and optionally an organic extractant. Said need includes organic phase treating or handling techniques or processing steps. Continuing upstream biocatalytic process innovation is driving the need for innovations in downstream processing of organic process streams containing biocatalytically produced product, instead of typical aqueous streams.

The present disclosure addresses this need. In particular, the present disclosure addresses this need for recovery of a biocatalytically produced substance from a fluid stream taken from a bioreactor system comprising both a reaction compartment wherein the biocatalytic production is carried out (typically in an aqueous reaction mixture) and a separation compartment wherein the fluid stream comprising the biocatalytically produced product is separated off from the reaction mixture. More in particular, the present disclosure provides a recovery process of a biocatalytically produced organic product from a fluid stream originating from a bioreactor system, wherein the fluid stream not only comprises a major amount of an organic phase, comprising the biocatalytically produced product and optionally one or more other organic substances (such as extraction solvent, side-products, unconverted organic substrate, organic nutrient)), but also minor amounts of an aqueous phase and of solid matter, such as biocatalyst material (which can comprise intact/living biocatalyst material or parts thereof, e.g. debris (such as cell walls), biological derived material (such as extracellular proteins), inorganic solid matter, solidified extractant, solidified (by)-product).

Typical biocatalytic separation processes, i.e. downstream processing, conventionally used in the art are water based processes, focussed on recovering substances in very low concentrations from large fractions of water, e.g. evaporation of water, chromatography, ultrafiltration. These processes are complex, cost intensive, energy intensive and generate waste streams, both as result of the technique used as well as the low concentration of substance to be recovered resulting in low driving force for separation. In an aspect, the present disclosure also addresses one or more of these drawbacks.

Accordingly, the present invention relates to a method according to any of the claims 1-27.

The method according to the disclosure and other features, aspects, and advantages of the apparatus, systems and methods of the present disclosure will become better understood from the following description, appended claims, and accompanying drawing wherein:
FIG 1A shows an example of centrifuged fluid stream (S0) with respectively solid content, water content and organic content layer, with low solids content
FIG 1B shows an example of centrifuged fluid stream (S0) with respectively solid content, water content and organic content layers, with higher solids content

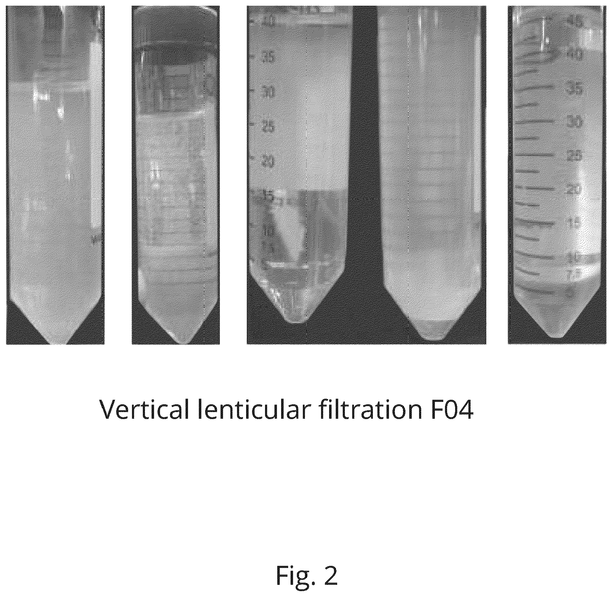

FIG 2 shows an example of liquid-liquid separation after solid content removal, illustrating the clear separation between aqueous phase and organic phase after removal of the solids in accordance with the method of the present disclosure.

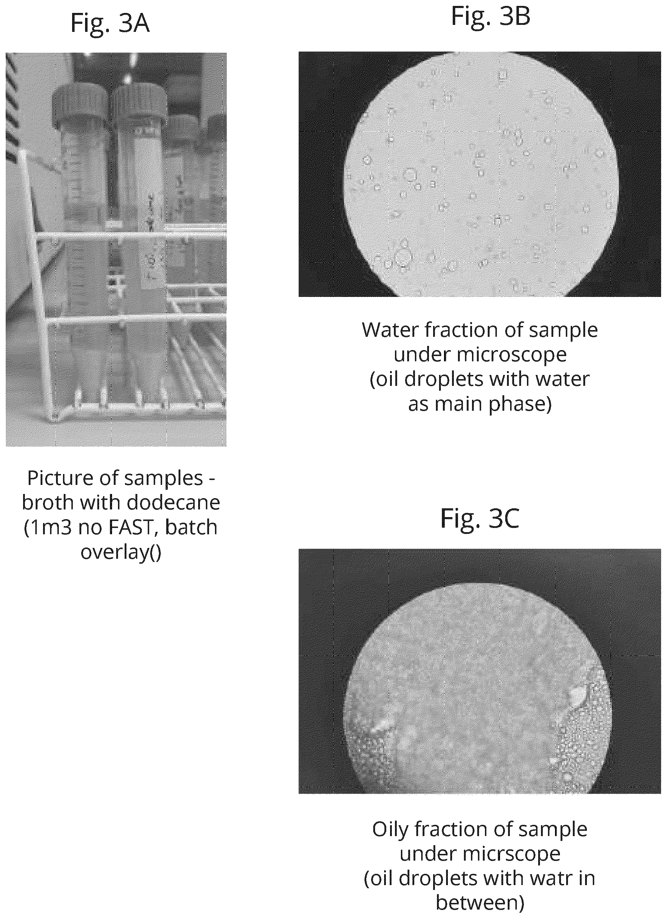

FIG 3A shows a picture of samples of fermentation broth with dodecane overlay (1m³ fermenter batch overlay) in which the organic liquid phase is colored red.
FIG 3B shows a picture of the water fraction of sample from FIG 3A under microscope showing organic phase droplets with water as main phase.
FIG 3C shows a picture of the organic phase fraction of sample from FIG 3A under microscope showing organic phase droplets with water in between.
FIGS4-9 are in particular useful aiding the skilled person in choosing particularly advantageous extractants and or back-extractants in a method according to the present disclosure.

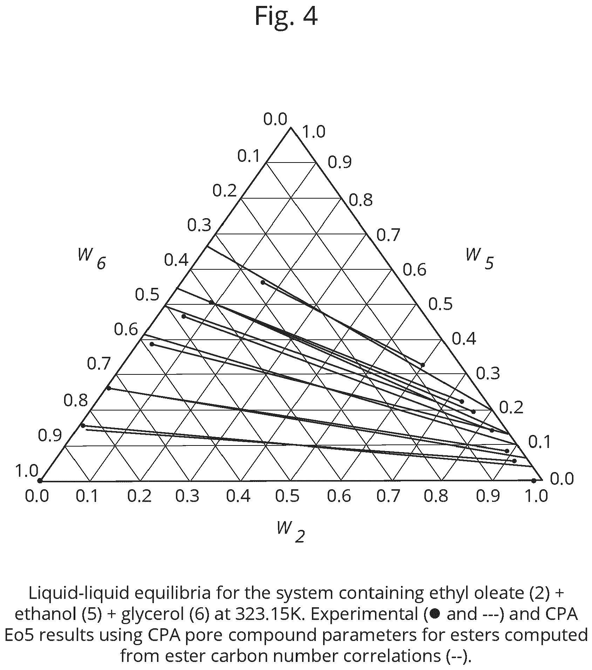

FIG 4 provides a schematic diagram for liquid-liquid equilibria for ternary systems containing esters, ethanol and glycerol at 323.15 K and 353.15 K.;

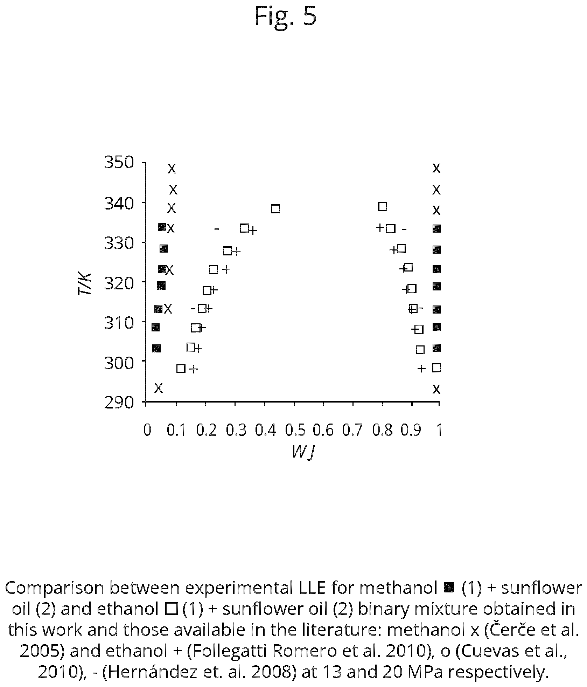

FIG 5 presents the liquid-liquid equilibrium curves for a binary mixture of (a) methanol and sunflower oil and (b) ethanol and sunflower oil;

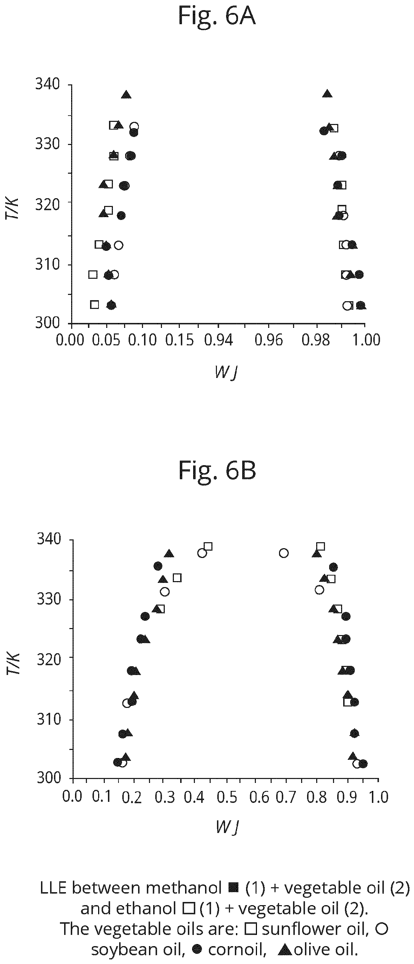

FIG 6 presents the liquid-liquid equilibrium curves for a binary mixture of (a) methanol and vegetable oil and (b) ethanol and vegetable oil The vegetable oils are sunflower oil, soybean oil, corn oil, and olive oil;

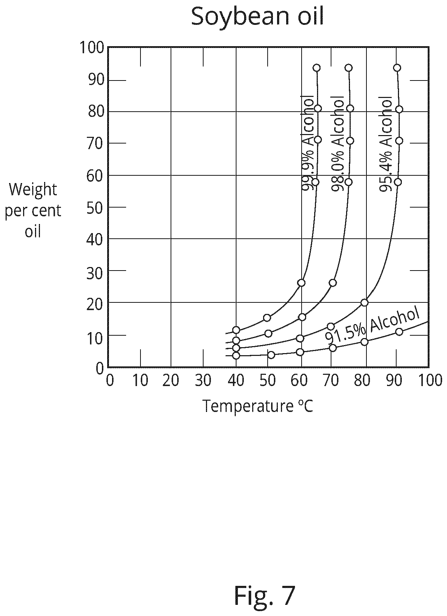

FIG 7 presents the weight percentages of soybean oil soluble in ethanol as a function of temperature;

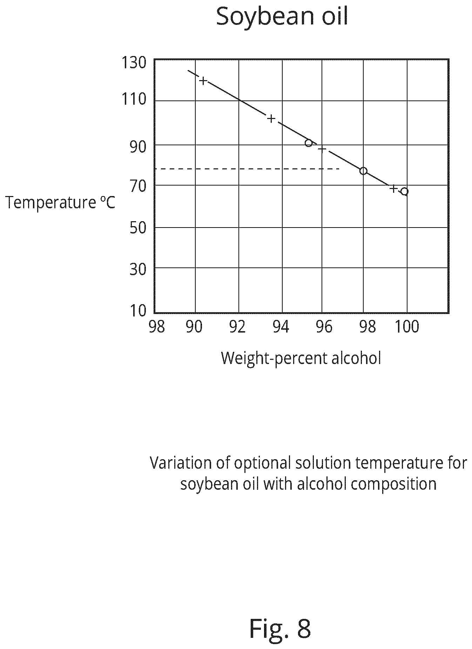

FIG 8 presents the variation of critical solution temperature for soybean oil with alcohol composition;

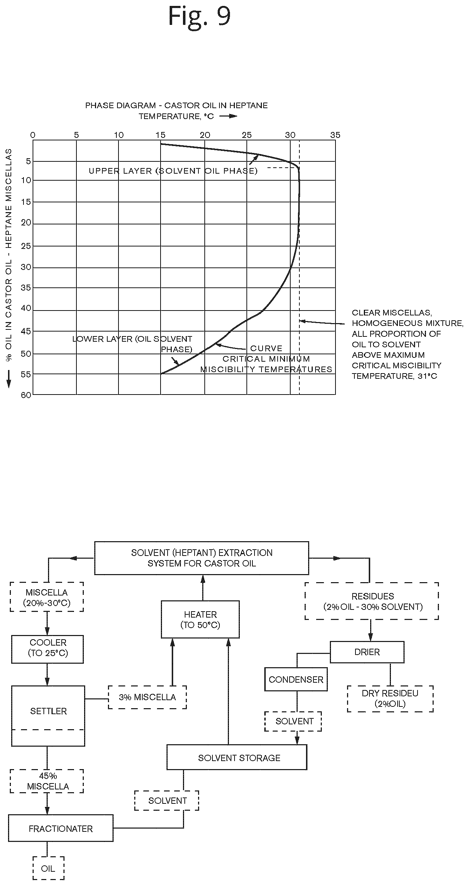

FIG 9 shows the phase diagram and separation process for castor oil extraction using heptane;

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise. The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

The term "(at least) substantial(ly)" or "essentially" is generally used herein to indicate that it has the general character or function of that which is specified. When referring to a quantifiable feature, this term is in particular used to indicate that it is at least 50 %, more in particular more than 75 %, even more in particular more than 90 % of the maximum that feature. The term `essentially free' is generally used herein to indicate that a substance is not present (below the detection limit achievable with analytical technology as available on the effective filing date) or present in such a low amount that it does not significantly affect the property of the product that is essentially free of said substance. In practice, in quantitative terms, a product is usually considered essentially free of a substance, if the content of the substance is 0 - 1 wt.%, in particular 0 - 0.5 wt.%, more in particular 0 - 0.1 wt.%.

In the context of this application, the term "about" means generally a deviation of 10 % or less from the given value, in particular a deviation of 5% or less, more in particular a deviation of 2% or less.

For the purpose of clarity and a concise description, features are described herein as part of the same or separate embodiments, however, it will be appreciated that the scope of the invention may include embodiments having combinations of all or some of the features described.

As used herein "organic" refers to any organic substance (such as biocatalytically produced product that is chemically oxidisable, as can be determined by the Chemical Oxygen Demand (COD) test, as described in ISO 6060:1989. The biocatalytically produced organic product can be any organic chemical substance or mixture of organic chemical substance that can be produced biocatalytically, in particular fermentatively, in particular any such substance or group of substances mentions in the prior art cited herein. The method of the present disclosure is generally particularly advantageous for the recovery of a biocatalytically produced substance having a limited solubility in water, due to the substance's hydrophobic or lipophilic nature. Typically the aqueous solubility is less than 85 g/l, in particular less than 10 g/l, preferably 0-1 g/l, at least at the temperature(s) at which it is contacted with the biocatalytic reaction mixture and/or at ambient temperature (25 degrees C). Advantageously, the biocatalytically produced product has a higher affinity for a lipid, such as a liquid triglyceride (in particular a vegetable oil like sunflower oil or castor oil) or a liquid hydrocarbon, such as hexane or the like, relative to water.

The organic substance, can be a substance having 1 carbon or more carbons and at least 1 hydrogen. Usually, the organic substance, in particular the biocatalytically produced product has at least 3 carbons, in particular at least 4 carbons, more in particular at least 5 carbons. The organic substance, in particular the biocatalytically produced product, can have over a 1000 carbons (such as in case of produced biopolymers. In an embodiment the organic substance has up to 100, up to 50, up to 30 , up to 25 carbons, up to 20 carbons, up to 15 carbons or up to 12 carbons.

Advantageously, the method according to the present disclosure can be used to produce one or more organic substances selected from the group consisting of hydrocarbons, in particular monoterpenes, sesquiterpenes, aromatic hydrocarbons; isoprenoids (terpenoids); organic acids, in particular C5-C24 fatty acids, more in particular C12-C20 fatty acids; alcohols, in particular alcohols having at least 4 carbon atoms; ketones, in particular ketones having at least 5 carbon atoms; aldehydes in particular aldehydes having at least 5 carbon atoms; cyclic carboxylic esters, in particular lactones; non-cyclic esters, in particular non-cyclic esters having at least 5 carbon atoms; lipids in particular glycerides; ethers; pyridines; imines; imides; amines; amino acids; and peptides. For further details of preferred organic substances that can in be produced in accordance with the invention, reference is made to the prior art cited herein, in particular WO2021/010822 page 23, line 27 till page 27, line 10 of which the contents are incorporated herein by reference.

Further, a liquid organic substance for extracting the produced product of interest forms a phase separate from the biocatalytic liquid phase in the bioreactor can be present in S0 or used in the method according to the present disclosure. Typically the aqueous solubility of an extractant is less than 85 g/l, in particular less than 10 g/l, preferably 0-1 g/l, at least at the temperature(s) at which it is contacted with the fermentation medium and/or at ambient temperature (25 ⁰C). Particularly suitable liquids for the product recovery phase are organic liquids, in particular hydrophobic organic liquids. These may in particular comprise one or more compounds selected from the group of hydrocarbons, organic acids, alcohols, ketones, aldehydes, cyclic carboxylic esters, non-cyclic esters, lipids, amines, including mixtures thereof. Preferred are alkanes, triglycerides and hydrophobic alcohols. Particularly preferred alkanes have 6 carbons (C6) or more, more preferably C7-C25, e.g. C7-C15. Specific examples of particularly suitable liquid alkanes are hexanes, heptanes, octanes, nonanes, decanes, dodecanes and pentacosane. The alkane may be linear or branched or cyclic. Good results have, amongst others been achieved with dodecane. Further, liquid hydrophobic alcohols are particularly suitable. Preferably the alcohol is selected from C8 alcohols and higher, more preferably C10-C20 alcohols. The alcohol may be linear or branched or cyclic. Specific examples of particularly suitable alcohols are an octanol, a decanol, a dodecanol, oleyl alcohol and isomeric mixtures thereof. Further, liquid triglycerides are particularly suitable, preferably vegetable oils. Particularly preferred examples are castor oil, sunflower oil and soy(bean) oil. The various examples of liquids for the recovery phase that are given are not or poorly soluble in water. Furthermore they typically show good biocompatibility, which is of interest when using a living cell for the biocatalysis and using these liquids in the bioreactor system.

The term 'biocatalytical(ly)' is generally known in the art to describe methods wherein at least one reaction step in the method is catalysed by a biological material or moiety derived from a biological source, for instance an organism or a biomolecule derived there from. The biocatalyst may in particular comprise one or more enzymes. The biocatalyst may be used in any form. In an embodiment, one or more enzymes are used isolated from the natural environment (isolated from the organism it has been produced in), for instance as a solution, an emulsion, a dispersion, (a suspension of) freeze-dried cells, as a lysate, or immobilised on a support. In an embodiment, one or more enzymes form part of a living organism (such as living whole cells). Particularly good results have been achieved with living cells, in particular with yeast cells and with bacterial cells. Processes wherein the biocatalyst comprises a living cells are also referred to in the art as 'fermentative' methods. The present method can be used for anaerobic processes, aerobic processes and anoxic processes. Advantageously, the biocatalyst comprises a micro-organism selected from the group of bacteria, archaea and fungi, preferably selected from the genera *Pseudomonas, Gluconobacter, Rhodobacter, Clostridium, Escherichia, Paracoccus, Methanococcus, Methanobacterium, Methanocaldococcus* , *Methanosarcina, Aspergillus, Penicillium, Saccharomyces, Kluyveromyces, Pichia, Candida, Hansenula, Bacillus, Corynebacterium, Blakeslea, Phaffia (Xanthophyllomyces), Yarrowia, Schizosaccharomyces, Zygosaccharomyces, Saccharopolyspora* and *Zymomonas* more preferably from the group of *Corynebacterium glutamicum, Escherichia coli, Bacillus subtilis* , *Bacillus methanolicus, Pseudomonas aeruginosa, Pseudomonas putida, Rhodobacter capsulatus, Rhodobacter sphaeroides, Paracoccus carotinifaciens, Paracoccus zeaxanthinifaciens, Saccharomyces cerevisiae, Saccharomyces pastorianus, Schizosaccharomyces pombe, Aspergillus nidulans, Aspergillus niger, Aspergillus oryzae, Blakeslea trispora, Penicillium chrysogenum, Phaffia rhodozyma* (*Xanthophyllomyces dendrorhous*), *Pichia pastoris, Yarrowia lipolytica, Saccharopolyspora spinosa and Zymomonas mobilis,* in particular from the group of *Escherichia coli, Pseudomonas aeruginosa, Pseudomonas putida, Saccharomyces cerevisiae, Saccharopolyspora spinosa and Zymomonas mobilis.* A biocatalyst selected from this group may in particular be used for the production of a substance according to the previous paragraph.

Differences between the traditional biocatalytic separation and recovery of the substance from the organic liquid stream in accordance with embodiments of the present disclosure include one or more of the following (1) Recovery from an organic stream versus aqueous stream with different physical properties like boiling and melting point, (2) Lower concentration, and for some species much lower, or even absence of typical by-products/contaminants, like acids, ethanol, salts, due to selective extraction and phase separation in accordance with the present disclosure, reducing the number of separation steps required and the complexity, (3) higher biocatalytical product concentration (in any one or more of S0, L1 and S1), resulting in higher driving force for separation (specifically in step c), (4) degree of freedom in selection of the organic liquid, both with respect to physical properties in relation to the biocatalytically produced product, as with respect to separation technique perspective, from an affinity/concentration perspective, to facilitate easy and more intensified downstream processing.

The inventors further realised that in accordance with the present disclosure it is possible to make advantageous use of separation processes based on techniques more typical to the chemical industry, e.g. distillation, extraction, crystallization, evaporation, precipitation. These separation processes are known in the state-of-the-art in the chemical industry, however are not standard to the biotech industry. Due to the nature of biocatalytic product streams the application of these separation processes is not straightforward. This is not straightforward: the inventors realised that this is due to the presence of a small fraction of water, solid and optionally gas in many (at least substantially) organic biocatalytic product streams. The inventors realized that the solids stabilize a certain fraction of water in the organic phase. Further, the biocatalytically produced product concentration in effluent streams from bioreactor systems is usually lower than in effluent streams from chemical industry unit operations, which may also be considered prejudicial to using downstream processing techniques from the chemical industry, like distillation. Furthermore the nature of many biocatalytically produced products and extractants itself is different which for instance can be more thermally sensitive.

The inventors realisation of the relevance of the presence of an aqueous phase (including remains of reaction mixtures, such as fermentation broth), solids (such as biocatalyst or remains thereof) and optionally gas in organic streams, containing biocatalytically produced product, taken from a bioreactor system, and the removal thereof. This realisation in accordance with the present disclosure, surprisingly allows advantageous use of separation processes for product recovery from an organic fluid stream originating from a biocatalytic process, which are technically mature, widely applied in chemical industry, suitable for continuous (stable) operation, resource efficient (e.g. energy, auxiliary materials and equipment). Moreover, at least in a number of embodiments existing infrastructure can be used in the transition from petrochemical to biobased production.

It is a further advantage of the present disclosure that it allows recovery of the biocatalytically produced product in an at least substantially continuously manner. An example of a substantially continuous operation is a method with intermittent product hold-up, e.g. in hold-up vessels. In particular good results are achieved with continuous or substantially continuous extraction and removal of biocatalytically produced product from a biocatalytic reactor system. In a preferred embodiment, there is an additional step for product purification after recovery of the product (in step b or step c).

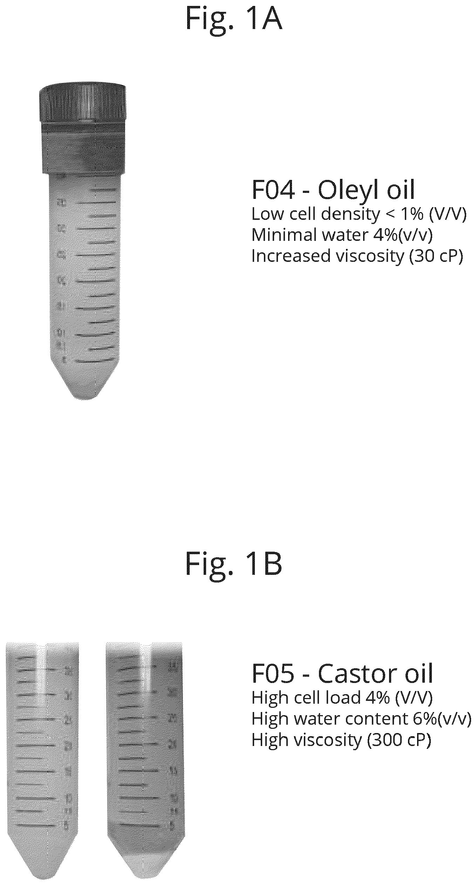

In the method of the present disclosure, fluid stream S0 originating from the biocatalytic reactor system comprises (i) an organic liquid phase L1, (ii) an aqueous liquid phase L2, and (iii) solids matter. The solids matter typically comprises, in particular at least substantially consists, of biocatalyst material, such as cellular material (cells, parts of cells, etc.) used for a fermentative production of the product. Solids material can be dispersed or suspended in L1, L2 or both in L1 and L2. Solids material may be present at an interface between L1 and L2. The solids material can be distinguished (and separated) from L1 and L2 in that a substantial fraction of the solid material does not pass a filter having a pore size of 0.2 micrometre. Thus a solid matter content can also be derived using such a filter. An content of L1 in S0 can be derived by centrifugation and/or decantation and/or evaporation of L2. A content of L2 in S0 can e.g. be determined by mass balancing after separation of L2, in lab often by centrifugation and/or after removal of solid content by filtration. Figures 1A and 1B illustrate how after centrifugation a sample of S0 separates in a L1 layer, a L2 layer and a solids layer. As can derived from the haziness of the sample solid matter and/or water liquid phase L2 is present in L1. Volumes can be determined and then weight % calculated from it or integral volumes can be weighed to obtain an amount of the fractions of L1, L2 and solid matter.

L1 and/or L2 can be present in the form of an emulsion or at a certain degree of emulsification in fluid stream S0. Further, the fluid stream optionally comprises a gas), which gas can be dissolved in the organic liquid, the aqueous liquid or form a separate gaseous phase.

The method of the present disclosure is used for recovery of biocatalytically produced product from a bioreactor effluent stream having a high content of organic phase (L1). Obtaining a fluid mixture containing the produced product with such high content (more than 50 wt.%), is generally not achieved with commonly employed fermentative processes, such as overlay fermentation, as the reaction mixture in a typical overlay fermentation contains up to 20% organic phase and not selectively withdrawn from the system. In present disclosure however the organic fluid stream S0 is derived from a biocatalytic system that has the capability of selectively withdrawn the organic phase obtaining a high organic content (more than 50 wt.%). Advantageously, the content of the organic liquid phase L1 is in the range of about 55 to about 99 wt.% based on total weight of the fluid stream S0, in particular in the range of 70-98.5 wt.%, based on total weight of the fluid stream S0, more in particular in the range of 75-98.0 wt.%, based on total weight of the fluid stream S0, even more in particular in the range of 80-97.5 wt.% based on total weight of the fluid stream S0.

Dependent on the biocatalytic production conditions and the biocatalytically produced substance, the organic liquid phase L1 may at least substantially consist of biocatalytically produced product. This is the case when the produced product is highly hydrophobic and is produced in sufficient amounts whereby it forms a sufficient volume of separate phase in the bioreactor system over time. Thus, in an embodiment, the biocatalytically produced organic product provides more than 50 wt. % of the organic substance content, preferably at least 70 wt. %, in particular at least 80 wt. %, more in particular at least 90 wt. % of the organic liquid phase L1. In practice, some other organic substances may still be present also when the product forms its own phase in the bioreactor system, e.g. side-products, organic matter used as substrate or nutrient for the biocatalyst, an organic auxiliary substance etc. Thus, in practice in such embodiment the biocatalytically produced product content in L1 will usually be more than 50 wt.% and less than 100 wt.%, in particular 99.9 wt.% or less, more in particular up to 99 wt.%, up to 98 wt. %, up to 97 wt.%, up to 96 wt. % or up to 95 wt.%.

In a further particularly advantageous embodiment, the organic liquid phase (L1) comprises an organic extractant solvent wherein the biocatalytically produced organic substance is present. The use of extractant(s) adds to flexibility in products that can be recovered effectively or efficiently, yet add complexity in recovery of the product from bioreactor effluents and thus a challenge in obtaining satisfactory effectivity (yield) or efficiency of the method. The present disclosure also addresses such challenge in particular. The relative amount of product in L1 when an extractant is used can vary widely. Usually the extractant forms the major component, when used. In particular, the extractant content can be in the range of 50 and 99.5 wt.% of L1, more in particular between 75 and 99 wt.% of L1, even more in particular between 85 and 99 wt.%. Usually, when an extractant is used, the biocatalytically produced product content can be in the range of 0.5 and 50 wt.% of L1, preferably between 1.0 and 25 wt.%, in particular between 1 and 15 wt.%. In a most preferred case the summed content of extractant and biocatalytically produced product is 100 wt.% or close to, in particular between 95 and 99.5 wt.%. The balance (up 100 wt.%) can be formed by organic side product and dissolved organic substance(s) from the reaction mixture (such as nutrient, side-product), etc..

Typically, in a method of the present disclosure the fluid stream (S0) is obtained in a device (apparatus) comprising a reaction section and a separation section and/or biocatalytic system that allows selective withdrawal of S0. Advantageously the reaction section and the separation section form a single device or part of a single device, which may also be referred to as an integrated bioreactor system, containing a reaction compartment and separate therefrom a separation compartment. Suitable integrated bioreactor systems can e.g. be based on PCT/NL2023/050445 or WO2021/010822, of which the contents - in particular the claims, Figures and figure descriptions of the devices - are incorporated by reference.

A method according to the present disclosure is particularly suited for product recovery processes from a fluid stream (S0) taken from an at least substantially continuous extractive fermentation. Such process is known in the art as such. Typically, in said extractive fermentation a product recovery phase comprising biocatalytically produced organic substance is separated from a reaction mixture wherein the product has been produced. Typically, in the extractive fermentation from which S0 is advantageously taken, a reaction mixture is provided, wherein the product is produced biocatalytically (using cells, such as micro-organisms like yeasts, bacteria, archaea, or cultures of cells from multicellular organisms). The reaction mixture comprises an aqueous phase, in which the biocatalyst is preferably dispersed or dissolved, which aqueous phase comprises a substrate for the biocatalyst. Further droplets of a fluid product recovery phase (comprising the produced product and optionally an extractant) are dispersed in the continuous aqueous phase, into which droplets produced product migrates. The product recovery phase comprising the produced product and the aqueous phase are subjected a separation, whereby a fluid stream enriched (compared to the contents of the reaction mixture in the reaction section) in organic phase (product recovery phase), comprising the produced product (and extractant if extractant is used), is obtained, but also some water and solid matter (in particular biocatalyst material, such as cells, cell parts etc) , and a fluid stream enriched in aqueous phase. The fluid stream enriched in organic phase is then taken as fluid stream S0 for processing in the method according to the disclosure. Production of the organic product and the separation of the product recovery phase are advantageously carried out in a single apparatus comprising a reaction section, containing the reaction mixture wherein the product is biocatalytically produced, and a separation section wherein the fluid stream enriched in organic phase comprising the produced product (and the extractant, if used) is separated from the aqueous phase. The advantageous at least substantially continuous extractive fermentation, comprises a simultaneous production and separation stage,
a) wherein at least during said simultaneous stage,
b) substrate and/or product recovery phase is fed into the reaction section continuously or intermittently,
c) flow conditions in the reaction section are usually turbulent flow conditions,
d) reaction mixture - of which mixture the product recovery phase comprises the produced product - is fed continuously or intermittently from the reaction section into the separation section, which fed reaction mixture preferably enters said separation section under essentially laminar flow conditions,
e) in which separation section the separation to obtain the fluid enriched in organic phase (providing S0) is separated from the aqueous phase, preferably under essentially laminar flow conditions or intermittently alternating between laminar flow conditions and no-flow conditions, and
f) the fluid enriched in organic phase (providing S0), comprising the biocatalytically produced product (and extractant, when used), is typically recovered continuously or intermittently from the separation section of the apparatus.
   The extractive fermentation is advantageously carried out based on a method described in PCT/NL2023/050445 or WO2021/010822, with the proviso that the process conditions are such that the fluid stream S0 that is taken from the bioreactor system contains solid matter and an aqueous liquid phase (L2), in addition to the organic liquid phase (L1) having a content of more than 50 wt.% of S0.

The method of the present disclosure also offers a possibility to preferentially remove damaged cells and other cellular material that has reduced desired biocatalytic activity or no significant desired biocatalytic activity anymore from a fermentative bioreactor system from which the fluid stream S0, comprising the biocatalytic product is taken. Thus, the present disclosure also allows to carry out fermentation under conditions that are more stressful to the biocatalyst (whereby cells may be e.g. damaged), yet that may be desired in some cases. E.g. higher turbulent conditions in the reaction mixture could cause increased shearing, yet smaller droplets of product recovery phase, thereby increasing interface area between product recovery phase and aqueous phase; e.g. varying temperature. Also other conditions can be chosen such that the ratio living biocatalytic cells compared to cell material having reduced or no significant biocatalytic activity is lower in S0 than in the aqueous phase of the reaction mixture. Thus, the method of the present disclosure can contribute to keeping the content of undesired material derived from the biocatalyst (dead cells, debris etc) in the reaction mixture in the bioreactor at favorably low levels.

The method for product recovery can be of particular benefit for product recovery from organic liquid streams, more specifically organic streams that are used for extraction of biocatalytically produced product or biocatalytically produced product(s) that form their own liquid phase and form the bulk component (more than 50 wt.%, based on total weight of the organic liquid phase). Even more specifically, in case of extraction, organic streams that are used for continuous extraction and removal of biocatalytically produced product from a biocatalytic reactor system. In a preferred embodiment the process for recovery of the biocatalytically produced product is continuous and there is an additional step for product purification.

Unlike typical downstream processing in biotechnology, downstream processing in accordance with the present disclosure is carried out with an enriched organic fluid stream (S0), comprising more than 50 percent in weight of organic liquid phase, advantageously originating directly from a biocatalytic reactor system, i.e. advantageously without further having been subjected to separation steps downstream of the separation section of the biocatalytic reactor system . In this stream there is a need for solid content and aqueous phase removal, as well as for product recovery.

Solid content removal has multiple benefits, such as (1) solids, e.g. cell material like proteins, stabilize emulsions and removing the solids content enhances liquid-liquid separation, (2) limiting biological activity, in case of living cell fermentation removal of solids content limits the biological activity 'zone', thereby also preventing biological based reactions, such as corrosion and/or other equipment and product integrity issues downstream (3) product purification, (4) prevention of fouling of downstream process equipment.

Solid content in the enriched organic fluid stream (S0) is typically at least 0.1 wt.%. Solid content in the enriched organic fluid stream (S0) is typically up to 5 wt%, preferably in the range of 0.1 - 4.5 wt.%, more preferably in the range of 0.5 - 4.0 wt% . Usually, 50-100 wt.%, in particular at least 80 wt.%, more in particular at least 90 wt.% of the solids content is composed of biocatalyst material, preferably cellular material, such as intact (living) cells, cell parts or a combination thereof. In comparison to typical fermentation processes the cell concentration in the broth and thus in the aqueous product stream is 50-100 g/L (~5 - 10 wt%). As the solids content is typically lower than typical aqueous product streams from biocatalytic processes this changes solids separation operation as the solid load per volume is different.

Also it was observed, in a preferred embodiment, that the solid matter removed in the fluid stream (S0) is relatively high with regard to water content. In such embodiment, the biocatalyst material, in particular cellular material, may be present in a content of more than 10 wt.% relative to the total water content in S0, in particular 15-50 wt.%, more in particular 20-40 wt.%, relative to the total water content in S0. It was found that a portion of this fraction is usually non-productive material, such as dead cell matter, which floats/goes to the water/organic surface/interface. This is different behaviour compared to the living active cells, which stay dispersed in the broth and do not, in significant quantities, go with the fluid stream (S0).

Aqueous phase removal take place in a preferred embodiment after solid content removal as it enhances the liquid-liquid separation. Aqueous phase removal has multiple benefits, such as (1) product purification, e.g. removing acids and salts via the aqueous phase, (2) prevention of fouling of downstream process equipment by for example salts (3) limiting corrosion issues downstream, e.g. as result of free water containing acids (4) preventing issues with distillation column as result of free water in the feed, e.g. slugs of free water can cause steam explosion and cause damage to distillation column internals.

The fluid stream (S0) from which product is recovered comprises an aqueous phase. Typically, the content of the aqueous liquid phase (L2) is less than 50.0 wt.% of the total weight of the fluid stream, preferably 45.0 wt% or less, more preferably 30 wt.% or less, in particular 20 wt.% or less, more in particular as low as 1 wt.%. Aqueous phase removal is different from traditional biocatalytic product streams as in traditional biocatalytic product streams the aqueous phase (broth) is the bulk phase (more than 50% in weight of the total fluid stream), whilst in the method according to the present disclosure the aqueous liquid phase is less than 50 wt.% of the fluid stream (S0), preferably 30 wt.% or less, more preferably 20 wt.% or less. The aqueous liquid phase (L2) content is lower than typical aqueous product streams from biocatalytic processes. This allows utilisation of the aqueous phase to enhance separation and purification, by for example an acid/base wash. Accordingly, good results are achieved with a method according to the present disclosure, wherein the fluid stream (S0) originating from a biocatalytic reactor is subjected to an acid wash and/or a base wash, optionally after (a) removal of solid matter. This offers an important advantage of the present method, compared to performing a base wash and/or an acid wash on the organic liquid phase L1 (after separating from the aqueous phase in step b; before or after removing solid matter) or on the organic fluid stream S 1 (after step c), because the aqueous phase L2 can help to wash one or more impurities from the organic phase, improving effectivity and/or efficiency.

Also it must be noted that the properties of the organic liquid phase start to govern the fluid stream properties, such as viscosity, changing techniques or equipment type suitable for phase separation.

Product recovery step c, is advantageously applied. It is usually applied at least if the biocatalytically produced product is not the bulk component (<50 wt.% of total weight) in the organic fluid stream S1. If the biocatalytically produced product is the bulk component S1 may be subjected to step c or directly further processed (e.g. purified) or used, if desired.

Product recovery (step c) preferably uses techniques that are more cost effective than commonly used recovery techniques from fermentation broths, sustainable and more typical to the chemical industry, like distillation, extraction, crystallization, evaporation, precipitation. A preferred distillation technique is vacuum distillation (from 0.005 to less than 1.0 bara), preferably molecular distillation, in particular short path, centrifugal or falling film evaporation under ultra deep vacuum (5-200 mbara).

Distillation is a preferred technique for product recovery from L1 in particular when L1 comprises an extractant, more in particular when S0 is obtained from an at least substantially continuous extractive fermentation. Distillation is particularly preferred when the organic fluid stream (S1) comprises less than 50 wt.% biocatalytically produced product and more than 50 wt.% of extracting solvent and the biocatalytically produced product is more volatile than the extracting solvent. Stripping with a gas, e.g. steam, is particularly useful for efficient recovery.

More preferably product recovery takes place by means of distillation if thermal stability of the product and the organic liquid substances, e.g. extractant, allow it and preferably when the product is more volatile than the bulk of the organic fluid stream (S1), e.g. extractant. In a preferred embodiment the distillation temperature is lowered by means of steam distillation and/or vacuum distillation and can even go to ultra deep vacuum, like molecular distillation. An advantage of steam stripping is its ease of condensation; released heat upon condensation in combination with a vacuum system also avoids problems with non-condensables. Although distillation is typical for the chemical industry it's not widely applied in biotechnology, especially not for lipophilic compounds, as due to their inhibiting nature they are typically present in trace amounts (0.1 - 1.0 g/L, i.e. -0.01-0.1 wt%, 100-1000 wppm)) in biocatalytic aqueous product streams and therefore regular distillation would not be feasible. With the organic fluid stream (S1) however the product concentrations are higher (often ~1.0 - 5.0 wt% when using an extractant, there can be exceptions on the higher side) than typical for biotechnology. Although often still lower than typical for chemical industry (10 wt% - 50 wt%). Thus, vacuum distillation or steam stripping can advantageously be employed for recovery of biocatalytically produced product from S1 wherein the content of biocatalytically produced product is up to 50 wt.%, in particular between about 1 and about 25 wt. %, more in particular between about 1 and about 15 wt. %; in a specifically preferred embodiment vacuum distillation is applied for recovery of biocatalytically produced product from S1 wherein the content of biocatalytically produced product is between about 1 and about 15 wt.%

In case distillation is not preferred or not an option, e.g. including amongst others, boiling point difference and/or cost and/or unstable or thermally sensitive product and/or bulk organic fluid stream (S1) and/or equilibrium limitation, the biocatalytically produced product can be recovered by extraction, in this case a back-extraction from the organic fluid stream (S1). The back-extraction comprises a Liquid-Liquid extraction step in which the product is recovered by an auxiliary liquid phase (L3). The product can subsequently be recovered from the auxiliary liquid phase by evaporation (of the auxiliary phase) and/or precipitation of the product and/or crystallization of the product.

In summary the biocatalytically produced product can be recovered by means of distillation and/or liquid-liquid extraction, but is not limited to these techniques. Also other techniques may be an option, such as evaporation and/or crystallization and/or precipitation and/or acidification and/or basification and/or a reaction like esterification and/or complexation. Of course due to the wide applicability range the product recovery step can be a combination and/or repeat of techniques in any order, depending on product and organic fluid stream/substances characteristics.

In a preferred embodiment there is also a step in which the product is further purified. Any of the techniques used for product recovery can also be used for purification, including distillation. But also techniques more typical to water based biotechnology downstream processing may be used, like chromatography, ultrafiltration. The method of the present disclosure has also the effect that the total volume of the organic fluid stream obtained after step c) is considerably smaller and more concentrated in product than the crude fluid stream taken from the bioreactor system. This contributes to efficiency and sustainability impact, also when using such techniques for further purification. Separation methodology choices to be made usually depends upon: type of separation process(es), order of separation steps, separation process setup (T,P,x,) if applicable extractant. Typical order of known separation processes: start with the easiest separations and preferably largest fractions/stream size reductions first. Easiest separations are fractions that are already in a different phase, e.g. solid, liquid, gas, at given conditions (T,P,x). After the easy separations the separations follow where a phase change is created or the desired compounds is transferred to another phase, either by changing the conditions of the system (T, P, other) or adding an auxiliary phase.

In case of product extraction, the organic product is typically in the same phase as the extractant, therefore not an easy phase separation. The simplest separation choice for product recovery from an organic phase is distillation. Typically this is beneficial when the product is more volatile than the organic phase and there is a sufficient difference in boiling point. If distillation is not possible due to non-volatile products and/or low difference in boiling point of substance and organic liquid or either the substance or organic liquid is thermally instable or for another reason distillation is not preferred/feasible, other separation techniques like extraction can be applied. Extraction is more complex than distillation as it requires an auxiliary liquid phase, which forms a 2 phase system with the organic liquid stream and has a sufficiently high affinity for the product. This makes it more complex, as the product now needs to be recovered from the auxiliary liquid phase. Preferred in case of extraction is to use a volatile extractant, which can be evaporated to allow for product recovery. Distillation and extraction are typical and extensively used product recovery techniques used in the chemical industry. Biocatalytic produced products however are typically more complex, as they contain numerous by-product and even in organic liquid phase are present in lower concentrations than typical in the chemical industry, and therefore even using these established separation techniques product recovery is not straightforward.

This disclosures enables product recovery from an organically enriched fluid stream (S0). As this stream, which in a preferred embodiment is typically higher boiling than water, comprises an organic phase, containing lower impurities and higher product concentration (than the biocatalytic medium/broth it's originating from), there are a number of benefits. These benefits include (1) lower energy requirement per product, (2) relatively smaller equipment size and lower number of unit operations resulting in lower capital requirement per product, (3) enabling more diverse DSP unit operations, e.g. more typical to chemical industry, (4) sustainability benefits, i.e. lower waste streams, lower energy requirement, (5) product integrity by non-water based process, different T, P, x, with early stage biocatalytic activity removal and substantially less water based catalytic conversion of product possible. Separation of solid matter can be performed in a manner known per se in particular by means of filtration, e.g. microfiltration, ultrafiltration, nanofiltration, membrane filtration, and/or centrifugation, e.g. disc stack centrifuge, decanter centrifuge, and/or flocculation. The inventors realised that the solid matter can have an adverse effect on the effective or efficient recovery or further purification of the biocatalytically produced product. The solid matter can e.g. stabilize emulsions, e.g. Pickering emulsion by proteins, and therefore the removal of a small fraction (solid matter) can aid the separation of a larger fraction (organic liquid phase).

In a particular preferred embodiment the solid content is separated first from S0 before separating the aqueous liquid phase. The removal of solid content before separation of the aqueous phase L2 aids the liquid-liquid phase (water - organic) separation. Removal of solid content, in particular solid matter comprising cell material, more in particular selected from cell debris, cell components, extracellular protein from cells, destabilises emulsions, dispersions, suspensions (if present), reduces the chance of formation of an emulsion, dispersion or suspension. Emulsions, dispersions or suspensions make it more difficult to remove aqueous phase or may adversely affect product recovery or further downstream purification. In accordance with the present disclosure water now more readily separates from the organic phase, often facilitating simple decanting. These preferred process configurations allows for improved liquid-liquid separation. In a next step the aqueous phase (L2) is separated from the fluid stream (S0), preferrably by means of gravity or centrifugal force, otherwise intermolecular force based techniques like adsorption , absorption, precipitation or surface force related coalescence and/or size exclusion and/or evaporation and/or separation based on polarity and/or chemical affinity by adding auxiliary components and/or changing pressure/temperature.

Further, the disclosure provides a method for product recovery by means of distillation, in particular a method for product recovery by means of stream distillation and/or vacuum distillation.

Steam distillation, preferred by steam (or other gas) stripping is typically used to lower the volatility and enhance the separation, in particular in case of relatively low product concentration. The volatility, e.g. vapor pressure, is effectively lowered by addition of an auxiliary gas component. This is especially beneficial in case of low product, and/or thermal sensitivity, concentrations to increase recovery.

Vacuum distillation, more preferably applied in case of high boiling organic extractant, e.g. vegetable oils ((refined) sunflower oil, castor oil, olive oil, palm oil, rapeseed oil, cotton seed oil, corn oil, linseed oil), or organic extractants having a similar or higher boiling point than vegetable oils, such as sunflower oil or castor oil, is especially beneficial in case the product and/or the organic extractant have thermal constraints, e.g. like a smoke point. Vacuum distillation is currently applied in purification of vegetable oils in their production process in order to remove impurities, but not in the use as extractant for the recovery of product.

Distillation can also be combined with (back)-extraction in case of low relative volatility, i.e. extractive distillation. Extractive distillation is different from extraction as it's involving V-L-L equilibria versus L-L equilibria and requiring a lower number of unit operations (thus capital).

In general with regard to distillation, any moisture still present in a small fraction and/or dissolved in the organic liquid stream (S1) is separated as well. In case water forms a two phase system with respectively the product and/or extractant (depending on properties and where the water goes in the distillation), water can be separated by settling (gravity/buoyancy). When there is not enough water to form a separate phase other techniques can be used, such as molecular sieve drying.

Further, the method in accordance with the present disclosure can make use of a single or multistep extraction process. In an advantageous embodiment in such method the biocatalytically produced product is recovered from the organic fluid stream (S1) by means of liquid-liquid extraction with an auxiliary liquid (L3) as back-extracting agent. Such method is particularly suitable also in case of an unstable or thermally sensitive product and/or bulk organic fluid stream (S1), and/or in case the product has a higher boiling product than the substance(s) from which it is to be separated, especially when the biocatalytically produced product is not the bulk of the organic fluid stream (S1), i.e. forms less than 50 wt.% of S1. The principle of back-extracting, rather than using the auxiliary liquid (L3) directly in the bioreactor, also allows the use of extractants that are not suitable a extractant for extraction of the biocatalytically produced product in the biocatalytic reactor for reasons as biocompatibility, volatility, emulsification behaviour with broth/water, miscibility with water or the like.

In a specially preferred embodiment, the biocatalytically produced product is recovered by a multistep extraction process, comprising
i. a first liquid-liquid extraction of the organic fluid stream (S1) with a first auxiliary liquid (L3) as back-extracting agent, resulting in organic liquid stream (S3) after liquid-liquid separation followed by
ii. a second liquid-liquid extraction of the organic fluid stream (S3) with a second auxiliary liquid (L4) as extracting agent.

This method is especially suitable also when the second auxiliary liquid (L4) is unsuitable as an extractant for extraction of the biocatalytically produced product in the biocatalytic reactor for one or more of the following reasons: biocompatibility, volatility, emulsification behaviour with broth/water, miscibility with water and the second auxiliary liquid L4 is not a suitable extractant as a first auxiliary liquid, for reasons of miscibility and/or compatibility.

In a preferred embodiment, L1 comprises an organic extractant used in the bioreactor system to recover biocatalytically produced product and in which L3 is more volatile than said extractant. In a particularly preferred embodiment, L1 comprises an organic extractant used in the bioreactor system to recover biocatalytically produced product and in which L4 is more volatile than said extractant and L3.

In a further preferred embodiment the product is recovered from the auxiliary liquid phase following the liquid-liquid extraction by evaporation and/or or wherein the product isolated by precipitation, in particular by crystallization, e.g. by reducing the temperature to a temperature at which the organic substance solidifies, whilst the back-extraction liquid remains liquid, and/or variations thereof.

In a further preferred embodiment L1 comprises an organic extractant used in the bioreactor system to recover biocatalytically produced product and said extractant in L1 is a vegetable oil, preferably selected from the group consisting of sunflower oil, canola oil, soybean oil, olive oil, palm oil, rapeseed oil, cotton seed oil, corn oil, linseed oil, including mixtures thereof and L3 is a short chain alcohol, preferably ethanol or methanol.

In a further preferred embodiment L1 comprises an organic extractant used in the bioreactor system to recover biocatalytically produced product and L1 is a vegetable oil, more specifically Castor Oil, and L3 is an alkane, preferably hexane.

In a further preferred embodiment L1 comprises an organic extractant used in the bioreactor system to recover biocatalytically produced product and said extractant in L1 is an ester, L3 is a vegetable oil and L4 is a short chain alcohol, preferably ethanol or methanol.

Liquid-Liquid extraction is at least a two step process by itself and includes at least a step where the two liquids are mixed to allow for transfer of the substance from one liquid to the other. And at least a step where the two liquids are separated from each other. Miscibility of the two liquids can be changed by changing P, T, x to enhance contact and/or separation. For example cooling of two extractant mixture creates a two phase system allowing separation of extractant and (co- or auxiliary) extractant.

For product recovery by extraction in particular, the inventors realized that it would be desirable to more specifically provide a method for product recovery from vegetable oils and/or esters. Inventors found that by combining vegetable oils and short chain alcohols, preferably ethanol or methanol, for most vegetable oils there is a temperature range where the mixture is miscible and there is a temperature where the mixture is not miscible. The invention enables the use of a water soluble extractant further in the process and expends the range of auxiliary species that can be used in product concentration and purification unit operations.

This can advantageously provide extraction of product from the vegetable oil and subsequent phase separation between the vegetable oil and the short chain alcohol.

Although an ester is typically miscible with short chain alcohols and not miscible with vegetable oils. Thus, in a preferred embodiment the ester is first brought into contact with the vegetable oil, before using the short chain alcohol to extract the product from the vegetable oil.

Especially also considering food application, the usage of biobased vegetable oils, short chain alcohols and/or extractants approved for food processing is preferred for such processes.

In a preferred embodiment in the single step back extraction L1 is a vegetable oil, preferably, sunflower oil, canola oil, soybean oil, olive oil, palm oil, rapeseed oil, cotton seed oil, corn oil, linseed oil, including mixtures thereof and L3 is a short chain alcohol, preferably ethanol or methanol. Or in another unique combination L1 is vegetable oil, more specifically Castor Oil and L3 is an alkane, preferably hexane.

In both the single step as two step extraction process the product can be recovered from the last extractant/auxiliary liquid phase by evaporation (of the auxiliary phase) and/or precipitation of the product and/or crystallization of the product.

In a preferred embodiment for the two step back extraction L1 is an ester, L3 is a vegetable oil and L4 is a short chain alcohol, preferably ethanol or methanol.

Many processes are enabled by means of extraction. The extraction process is possible as long as there is a liquid-liquid boundary line in the phase diagram of both liquid 1 and liquid 2 species that changes/shifts significantly in the range of temperature -10 to 50°C.

More specifically in an embodiment, starting with product in a (vegetable) oil, using a short chain alcohol (e.g. ethanol, methanol) as back-extraction solvent under low temperature conditions (0-20 °C).

Similar the phase behavior for the production of castor oil purification has a L-L phase separation window close to fermentation and ambient conditions. Modest cooling step will allow L-L phase separation. For a ternary component present in a mixture of these solvents, partition and phase separation will allow recovery of ternary component from the solvent of choice. Selection by means of affinity study of ternary component to both solvents is possible and will allow selection of liquid pairs enabling both extraction from broth by primary/first solvent, while also having an auxiliary/2^{nd} solvent that can extract the ternary (product) component from the first solvent with adequate efficiency.

## Claims

1. Method for the recovery of a biocatalytically produced organic product from a fluid stream (S0) originating from a biocatalytic reactor, which fluid stream (S0) has been separated off from a reaction mixture wherein the biocatalytically produced product has been produced, the fluid stream comprising
(i) an organic liquid phase (L1), said organic liquid phase comprising the biocatalytically produced product;
(ii) an aqueous liquid phase (L2);
(iii) the fluid stream comprising solid matter (SM);
the organic liquid phase (L1) content being more than 50 wt.%, based on total weight of the fluid stream (S0);
the method comprising
(a) removing solid matter from the fluid stream (S0), the organic liquid phase (L1), the aqueous liquid phase (L2) or from an organic fluid stream (S1) obtained in (b);
(b) separating the aqueous liquid phase (L2) at least substantially off from the fluid stream (S0) before, during or after (a) removing solid matter or from the organic liquid phase (L1), thereby forming an organic fluid stream (S 1), which organic fluid stream (S1) is enriched in biocatalytically produced product, compared to the fluid stream (S0) originating from the biocatalytic reactor; and
(c) if desired, after removing solid matter and separating the aqueous liquid phase (L2) at least substantially from the organic liquid phase (L1), recovering the biocatalytically produced product from the organic fluid stream (S1).

2. Method according to claim 1, wherein the fluid stream (S0) is obtained in an (integrated) apparatus comprising a reaction section, which may be a reaction zone, containing the reaction mixture wherein the product is biocatalytically produced, which reaction mixture comprises an aqueous phase and an organic product recovery phase, comprising the biocatalytically produced product, the apparatus further comprising a separation section, which may be a separation zone, wherein the reaction mixture is subjected to a separation, thereby forming the fluid stream (S0) enriched in biocatalytically produced product, compared to the reaction mixture and a further stream.

3. Method according to claim 1 or 2, wherein the biocatalytically produced organic product provides more than 50 wt. % of the organic substance content, preferably at least 70 wt. %, in particular at least 80 wt. %, more in particular at least 90 wt. %.

4. Method according to claim 1 or 2, wherein the organic liquid phase (L1) comprises an organic extractant solvent wherein the biocatalytically produced organic substance is present.

5. Method according to any of the preceding claims, wherein the content of L1 is in the range of 55-99 wt.% based on total weight of the fluid stream S0, preferably in the range of 70-98.5 wt.%, based on total weight of the fluid stream S0, more preferably in the range of 75-98.0 wt.%, based on total weight of the fluid stream S0, even more preferably in the range of 80-97.5 wt.% based on total weight of the fluid stream S0.

6. Method according to any of the preceding claims wherein, the fluid stream (S0) originating from a biocatalytic reactor is subjected to an acid wash and/or a base wash, optionally after (a) removal of solid matter.

7. Method according to any of the preceding claims wherein the biocatalytically produced product is recovered at least substantially continuously.

8. Method according to any of the preceding claims wherein (a) solid matter is removed from the fluid stream (S0) before separating the aqueous liquid phase (L2), the solid matter comprising cell material, in particular selected from cell debris, cell components, extracellular protein from cells.

9. Method according to any of the preceding claims wherein the fluid stream (S0) is taken from a biocatalytic reactor system,
in which biocatalytic reaction system the product is biocatalytically produced in a reaction section, which may be a reaction zone, containing a reaction mixture comprising an aqueous reaction phase and biocatalyst, which biocatalyst comprises a cellular catalyst, in particular living cells, the reaction mixture further comprising an organic product recovery phase, wherein the fluid stream (S0) is formed in a separation section, which may be a reaction zone of the biocatalytic reactor system, said formation comprising a phase separation of the reaction mixture into the fluid stream (S0) enriched in biocatalytically produced product and a stream enriched in aqueous reaction phase, wherein fluid stream S0 contains remains of the cellular catalysts, in particular cell debris and the content of the remains of the cellular catalysts in the fluid stream S0, relative to the aqueous phase content (L2) is higher than the content of the remains of the cellular catalysts in the aqueous phase of the reaction mixture present in the biocatalytic reactor.

10. Method according to any of the preceding claims wherein the solid matter is removed by means of filtration, e.g. microfiltration, ultrafiltration, nanofiltration, membrane filtration, and/or centrifugation, e.g. disc stack centrifuge, decanter centrifuge, and/or flocculation.

11. Method according to any of the preceding claims wherein (b) the aqueous phase (L2) is separated by means of gravity or centrifugal force, and/or intermolecular force based techniques, like adsorption, absorption, precipitation or surface force related coalescence and/or size exclusion and/or evaporation and/or separation based on polarity and/or chemical affinity by adding auxiliary components and/or changing pressure/temperature.

12. Method according to any of the preceding claims wherein in step (c) the biocatalytically produced product is recovered from the organic substance(s) in the organic fluid stream (S 1) by means of liquid-liquid extraction and/or evaporation and/or distillation and/or crystallization and/or precipitation and/or acidification and/or basification and/or a reaction like esterification and/or complexation in any order of combination thereof.

13. Method according to any of the preceding claims, wherein the fluid stream (S0) is taken from a biocatalytic reactor system,
in which biocatalytic reactor system the product is biocatalytically produced in a reaction section, which may be a reaction zone, containing a reaction mixture comprising an aqueous reaction phase, biocatalyst, and an organic product recovery phase, which product recovery phase comprises the biocatalytically produced product and an extracting solvent in which biocatalytic reactor system the fluid stream (S0) is formed in a separation section, which may be a reaction zone, of the biocatalytic reactor system, said formation comprising a phase separation of the reaction mixture into the fluid stream (S0) enriched in biocatalytically produced product and a stream enriched in aqueous reaction phase,
wherein the biocatalytically produced product is recovered from the organic fluid stream (S1) by means of distillation.

14. Method according according to claim 13, wherein the organic fluid stream (S 1) comprises less than 50 wt.% biocatalytically produced product and more than 50 wt.% of extracting solvent and the biocatalytically produced product is more volatile than the extracting solvent.

15. Method according to any of the preceding claims, wherein the product is recovered from the organic fluid stream (S1) by steam stripping or stripping with another gas.

16. Method according to any of the preceding claims, wherein the product is recovered from the organic fluid stream (S1) by vacuum distillation (0.005 - 1.0 bara), preferably molecular distillation, in particular short path, centrifugal or falling film evaporation under ultra deep vacuum (5-200 mbara).

17. Method according to claim 16, wherein the organic fluid stream (S 1) contains a high boiling organic extractant, such as a vegetable oil or an extractant having about the same or higher boiling point as a vegetable oil.

18. Method according to any of the preceding claims, wherein the biocatalytically produced product is recovered from the organic fluid stream (S1) by means of liquid-liquid extraction with an auxiliary liquid (L3) as back-extracting agent.

19. Method according to claim 18, in which the biocatalytically produced product is recovered by a multistep extraction process, comprising
i. a first liquid-liquid extraction of the organic fluid stream (S1) with a first auxiliary liquid (L3) as back-extracting agent, resulting in organic liquid stream (S3) after liquid-liquid separation followed by
ii. a second liquid-liquid extraction of the organic fluid stream (S3) with a second auxiliary liquid (L4) as extracting agent.

20. Method according to claim 19, herein the second auxiliary liquid (L4) is unsuitable as an extractant for extraction of the biocatalytically produced product in the biocatalytic reactor for one or more of the following reasons: biocompatibility, volatility, emulsification behavior with broth/water, miscibility with water and the second auxiliary liquid L4 is not a suitable extractant as a first auxiliary liquid, for reasons of miscibility and/or compatibility.

21. Method according to claim 18, wherein the organic liquid phase (L1) comprises an organic extractant used in the bioreactor system to recover biocatalytically produced product and in which the auxiliary liquid (L3) is more volatile than said organic extractant.

22. Method according to claim 19 or 20, wherein the organic liquid phase (L1) comprises an organic extractant used in the bioreactor system to recover biocatalytically produced product and in which the second extractant (L4) is more volatile than said extractant and the first auxiliary liquid (L3).

23. Method according to any of the claims 18-22, in which the biocatalytically produced product is recovered from the auxiliary liquid phase following the liquid-liquid extraction by evaporation and/or precipitation and/or (freeze) crystallization and/or variations thereof.

24. Method according to any of the claims 18 or 21 or 23 in which the organic liquid phase (L1) comprises an organic extractant used in the bioreactor system to recover biocatalytically produced product and said extractant in the organic liquid phase (L1) is a vegetable oil, preferably selected from the group consisting of sunflower oil, canola oil, soybean oil, olive oil, palm oil, rapeseed oil, cotton seed oil, corn oil, linseed oil, including mixtures thereof and the auxiliary liquid (L3) is a short chain alcohol, preferably ethanol or methanol.

25. Method according to any of the claims 18 or 21 or 23, in which the organic liquid phase (L1) comprises an organic extractant used in the bioreactor system to recover biocatalytically produced product and the organic liquid phase (L1) is a vegetable oil, preferably Castor Oil, and the auxiliary liquid (L3) is an alkane, preferably hexane.

26. Method according to any of the claims 18-20 or 23 in which the organic liquid phase (L1) comprises an organic extractant used in the bioreactor system to recover biocatalytically produced product and said extractant in the organic liquid phase (L1) is an ester, the first auxiliary liquid (L3) is a vegetable oil and the second auxiliary liquid (L4) is a short chain alcohol, preferably ethanol or methanol.

27. Method according to any of the preceding claims, wherein the fluid stream (S0) originating from a biocatalytic reactor has a water content of more than 1.1 wt.%, based on total weight of fluid stream (S0), preferably in the range of 1.5 wt.% to 50 wt.% based on total weight of fluid stream (S0), in particular in the range of 2.0 wt.% to 30 wt.% based on total weight of fluid stream (S0), more in particular in the range of 2.5 wt.% to 20 wt.% based on total weight of fluid stream (S0).
